# EUROPEAN PATENT SPECIFICATION

(11) **EP 3 235 535 B1**
(45) Date of publication and mention of the grant of the patent: **03.04.2019**
(21) Application number: 16183447.8
(22) Date of filing: 09.08.2016
(51) Int. Cl.: A61M 16/06, A61M 16/08

(54) **NASAL HIGH FLOW THERAPY DEVICE**
VORRICHTUNG FÜR NASALE HIGH-FLOW-THERAPIE
DISPOSITIF DE THÉRAPIE NASALE À FLUX INTENSE

(30) Priority: 21.04.2016 US 201662325727 P
(43) Date of publication of application: 25.10.2017
(73) Proprietor: L'AIR LIQUIDE, SOCIETE ANONYME POUR L'ETUDE ET L'EXPLOITATION DES PROCEDES GEORGES CLAUDE, 75007 Paris (FR)
(72) Inventor: Boulanger, Thierry, PA 19081 (US)
(74) Representative: Pittis, Olivier

(56) References cited:
- EP-A1- 2 233 167
- EP-A1- 2 720 005
- WO-A1-98/18513
- WO-A1-2015/156690
- WO-A2-2009/064202
- WO-A2-2012/037469
- FR-A1- 2 827 778
- US-A- 4 989 599
- US-A1- 2009 101 147
- US-A1- 2014 166 009

## Description

### Background

The invention concerns a nasal sub-system comprising two prongs that are inserted into the patient's nostrils for delivering a respiratory gas to a patient in need thereof, a gas delivery assembly including such a nasal sub-system and a measurement module, and a gas delivery apparatus comprising a high flow generator for delivering a respiratory gas to a patient by means of such a gas delivery assembly.

Nasal High Flow Therapy or NHFT is a widespread therapy used in many hospitals, especially in acute care services, against respiratory distresses. It does not require any mechanical ventilation and it can help some patients avoid being intubated.

Basically, NHFT consists in providing a humidified, high flow of respiratory gas to a patient through a nasal cannula. Gaseous flows typically range from 10 to 60 L/min for adults and 1 to 10 L/min for infants.

The nasal cannula comprises two prongs that are inserted into the patient's nostrils, although not totally occluding them. The prongs deliver the gaseous flow that the patient inhales and the space between the outer portion of the prongs and the nostrils helps the expired gaseous flow to be vented upon exhalation.

NHFT provides many advantages compared to continuous oxygen treatments or even to non-invasive ventilation, e.g.:
- the gaseous flow can be enriched with Oxygen (O₂) up to 100%, i.e. the respiratory gas can be composed of air, (O₂-enriched air (>22 vol% of O₂) or pure O₂;
- the set flow can be greater than the inspiratory peak flow of the patient so as to limit his work of breathing, thereby assisting in the avoidance of fatigue of respiratory muscles.
- the flow helps by flushing out CO₂ from the dead space of the upper airways, thereby enhancing the ventilation.
- the flow and the space between the prongs and the nostrils create a positive pressure, which exhibits a beneficial action on ventilation.
On top of that, this therapy is extremely simple as:- only a flow and a level of oxygen have to be set by the physician or similar;- the nasal cannula is easy to be put in place; and- the patient can speak and even eat, while receiving the therapy.
However, today, medical service providers do not have easy access to parameters that might help them in setting the right gas flow and gas concentration for obtaining the best therapy for a given patient. Instead, they usually rely only on oxygen saturation (i.e. SpO₂).
For instance, in some cases, a physician may decide to increase the O₂ concentration for reaching an adequate O₂-saturation in the blood of the patient, whereas increasing the flow of gas would have been a better solution. In other instances, a physician may decide to provide a higher flow of gas, but such a high flow may produce significant pressure levels that can be detrimental over time for some patients. In that case, increasing the O₂ concentration in lieu of the gas flow might be a better solution.
Furthermore, it happens that some patients under NHFT persist in their respiratory distress and have to be intubated eventually, despite the NHFT treatment. One indicator of a possible future intubation is a persistent dyspnea despite the NHFT therapy or modification of the minute volume ventilation; however, this indicator is not easily assessed by medical service providers.
It is clear that giving access to parameters such as the intra-nasal pressure, minute ventilation and respiratory rate of patients under NHFT would be valuable for medical service providers.

WO2015/156690 A1 discloses a respiratory therapy system configured to deliver gases to a patient, the system having a non-sealed gas flow generating arrangement configured to deliver a high flow of positive gas to an airway of a patient and a negative flow of gas away from an airway of the patient. The positive and negative flows of gas can be generated simultaneously. The system comprises nasal prongs comprising first and second gas passageways received in a single conduit interfacing with a manifold that split the positive and negative flows using a pair of barriers that extend into the prongs.The flows are split in such a way that both prongs are pneumatically linked with both the positive and negative flows.

Similar arrangements are known from WO98/18513 A1, WO2012/037469 A2 and US4989599 A.

### Summary

It is one purpose of the present invention to provide additional parameters to medical service providers, helping them to optimize the treatment dosing and administration parameters and to improve the care delivered to the patient, potentially avoiding intubation or reducing the length of stay.

One solution according to the present invention concerns a gas delivery assembly as defined in the appended claim 1.

Depending on the embodiment, a gas delivery assembly according to the present invention can comprise one or more of the following features:
- the vent conduit of the hollow body of the nasal sub-system is in fluid communication with the internal chamber of the hollow body via each pair of inner channels;
- an internal separation wall arranged in each prong of the nasal sub-system separates the first passage from the second passage of each prong;
- the nasal prongs of the nasal sub-system are sized so as to match the inner walls of the patient's nostrils;
- the prongs of the nasal sub-system have a conical or tronconical general shape;
- the inlet port of the supplementary conduit of the nasal sub-system is located in the vicinity of the free end of one of the nasal prongs.
- the supplementary conduit of the nasal sub-system comprises an outlet port located in the vicinity of the venting port of the vent conduit;
- the nasal sub-system further comprises a fixing system for maintaining the nasal prongs in position into the patient's nostrils.
- the main module body is attached to the nasal sub-system.

Another embodiment of the present invention concerns a gas delivery apparatus comprising a high flow generator for delivering a respiratory gas to a patient and a gas delivery assembly according to the present invention, as described hereabove.

Depending on the embodiment, a gas delivery apparatus according to the present invention can comprise one or more of the following features:- it further comprises a processing unit fluidly connected to the nasal sub-system.- it further comprises a processing unit electrically connected to the high flow generator.- it further comprises a processing unit comprising two pressure sensors electrically connected to a controller for providing pressure signals to said controller.- the processing unit further comprises a visual interface electrically connected to the controller for displaying data issued by the controller.- the processing unit further comprises a visual interface such as a screen or display.

### Brief Description of the Drawings

Other features, aspects and advantages of the present invention will become apparent from the following detailed description when taken in conjunction with the accompanying drawings which illustrates, by way of examples, the present invention, among which:
- Figure 1 is a schematic representation of a gas delivery assembly comprising a nasal interface according to the prior art,
- Figure 2 is a schematic representation of the nasal interface of Figure 1,
- Figure 3 represents an embodiment of a gas delivery assembly comprising a nasal interface according to the present invention,
- Figure 4 represents an embodiment of a nasal interface of a gas delivery assembly according to the present invention,
- Figure 5 represents a supplementary module of a gas delivery assembly that is directly attached to the nasal interface of Figure 4,
- Figure 6 represents an embodiment of a processing unit of the gas delivery assembly of Figure 3, and
- Figure 7 represents a supplementary module of a gas delivery assembly that is remotely connected to the nasal interface of Figure 4.

### Description of Preferred Embodiments

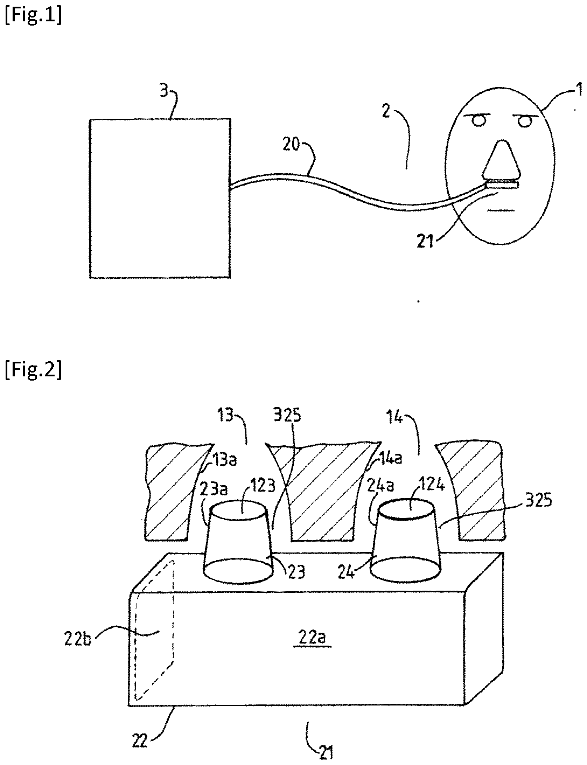

Figure 1 shows a gas delivery equipment comprising a cannula assembly **2** according to the prior art that fluidly connects a patient **1** to a high flow generator **3,** such as the device called Precision Flow™ distributed by Vapotherm or the device called Optiflow™ distributed by Fisher & Paykel.

The cannula assembly **2** comprises an elongated flexible tubing **20,** such as a flexible hose, comprising, at one end, a nasal sub-system **21** connected to the nose of patient **1** thereby delivering respiratory gas to the patient **1.** The elongated flexible tubing **20** is connected, by its other end, to the high flow generator **3.**

As detailed in Figure 2, the nasal sub-system **21** comprises a hollow body 22 with an inner chamber **22a,** acting as a manifold for receiving the respiratory gas conveyed and fed by the flexible tubing **20,** and nasal prongs **23, 24,** i.e. little nozzles, in fluid communication with the lumen of the inner chamber **22a** of the hollow body **22,** that are inserted, in use, into the nostrils of the patient **1** for delivering a respiratory gas to the patient **1** in need thereof.

Each nasal prong **23, 24** comprises a unique inner channel or passage **123, 124** for conveying the gas from the inner chamber **22a** of the hollow body **22** to the nostrils **13, 14** of the patient **1.**

The hollow body **22** can have various shapes, for example be of circular or rectangular cross section. The gas fed by the tubing **20** travels successively in the chamber **22a** of the hollow body **22** and then in each nasal prongs **23, 24,** and eventually is distributed into the nostrils **13, 14** of the patient.

With such a prior art equipment, a gas tight connection/insertion of the prongs **23, 24** into the nostrils **13, 14** is not ensured as the outer peripheral walls **23a, 24a** of the nasal prongs **23, 24,** respectively, are not operated in a manner that provides a sealed contact with the inner walls **13a, 14a** of the nostrils **13, 14,** respectively. This means that a spacing **325** always exists between the inner walls **13a, 14a** of the nostrils **13, 14,** and the outer peripheral walls **23a, 24a** of the nasal prongs **23, 24,** respectively, leading to gas loses.

The gaseous flow delivered by the high flow generator **3** enters into the inner chamber **22a** of the hollow body **22** and is then directed to the prongs **23, 24** so that upon inhalation, only a part of the gaseous flow is inspired by the patient **1,** as the rest is unfortunately vented to the atmosphere, while escaping through the spacing **325** that inevitably exists.

In opposite, upon expiration, both gaseous flows coming, on the one hand, from the high flow generator **3** and, on the other hand, exhaled by the patient do circulate in said spacing **325,** thereby creating a positive expiratory pressure before being vented to the atmosphere.

Such architecture is not ideal as it prevents the use of gas mixtures including noble gases such as Helium due to cost issues due to significant losses of Helium or other medicinal gaseous or vaporous contents in the gas feed.

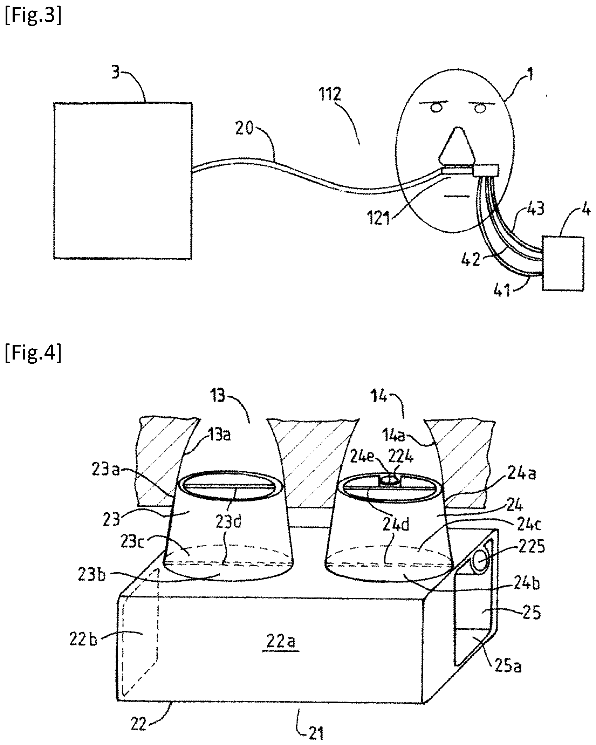

Figure 3 represents a first embodiment of a gas delivery assembly comprising a first embodiment of a cannula assembly **112** according to the present invention for fluidly connecting a patient **1** to a high flow generator **3** as in Figure 1.

As in Figures 1 and 2, the cannula assembly **112** comprises an elongated flexible tubing **20** connected, on the one hand, to the high flow generator **3,** and, on the other hand, to a nasal sub-system **121** inserted into the nose of patient **1,** thereby delivering respiratory gas to the patient **1.**

According to the present invention, the nasal sub-system **121** comprises further measurement devices for measuring and transmitting physiological signals, such as gas pressure and/or flowrate, to a processing unit **4.** These additional measurement devices comprise three measurement lines, namely flexible tubings **41, 42, 43** that are fluidly connected to the nasal sub-system **121** as shown in Figure 3.

Figure 4 shows an embodiment of a nasal sub-system **21** according to the present invention. It comprises, as in Figure 2, a hollow body **22** with an inner chamber **22a** and a gas inlet **22b** for receiving a respiratory gas fed by the flexible tubing **20** and a pair of prongs **23, 24** in fluid communication with the inner chamber **22a** of the hollow body **22** for delivering gas to the patient's nostrils **13, 14.**

Here also, the hollow body **22** can have any suitable shape, for example have a circular or rectangular section.

In this embodiment, the nasal prongs **23, 24** that are inserted in the patient's nostrils **13, 14** have been designed, i.e. sized and/or dimensioned, so as to exhibit a larger diameter and hence better match the inner walls **13a, 14a** of the patient's nostrils **13, 14,** thereby removing or minimizing the spacing **325** between the peripheral walls **23a, 24a** of the prongs **23, 24** and the inner walls **13a, 14a** of the nostrils **13, 14.** With such enlarged prongs **23, 24,** the gas tightness is improved compared to the embodiment of Figure 2 as the peripheral walls **23a, 24a** of the prongs **23, 24** are in contact with the inner walls **13a, 14a** of the nostrils **13, 14,** i.e. as no or almost no spacing **325** exists anymore in-between. Preferably, the prongs **23, 24** have a conical or tronconical general shape as shown in Figure 4 so as to better fit the inner shape of the nostrils **13, 14** and further to facilitate the insertion of the prongs **23, 24** into said nostrils **13, 14.**

In the embodiment of Figure 4, the nasal prongs **23, 24** comprise each two inner channels or passages **23b, 23c, 24b, 24c** arranged in parallel for directing the gas flows during inspiration and expiration phases.

In each prong **23, 24,** a first passage **23b, 24b** fluidly connects the internal chamber **22a** of the hollow body **22** with a nostril **13, 14** of the patient **1,** whereas a second passage **23c, 24c** fluidly connects the nostril **13, 14** with a vent conduit **25** arranged in the hollow body **22,** which is in fluid communication with the atmosphere, via one or several venting port **25a.** The vent conduit **25** does not communicate directly with the internal chamber **22a** of the hollow body **22.**

The first passage **23b, 24b** and the second passage **23c, 24c** of each prong **23, 24** is obtained in vertically separating each nasal prong, i.e. each little nozzle, by an internal separation wall **23d, 24d,** i.e. an inner thin wall separates each prong in two parts in the vertical way as shown in Figure 4.

With such a configuration, the gaseous flow, such as air, conveyed by the tubing **20** enters into the internal chamber **22a** of the hollow body **22,** travels through the first channels **23b, 24b** and is then delivered by the pair of prongs **23, 24** to the nostrils **13, 14** of the patient **1.**

During the inhalation phases of the patient **1,** a part of the gaseous flow is inspired by the patient **1,** whereas the rest of the gaseous flow is directed to the second channels **23c, 24c,** which are fluidly connected to the vent conduit **25** thereby allowing this excess of gas escaping to the atmosphere by venting port(s) **25a.**

In contrast, during the exhalation phases of the patient **1,** both gaseous flows coming from the internal chamber **22a** of the hollow body **22,** on the one hand, and exhaled by the patient, on the other hand, pass through the second channels **23c, 24c** and are vented to the atmosphere thanks to the vent conduit **25** and venting port **25a,** thereby creating a positive expiratory pressure or PEP.

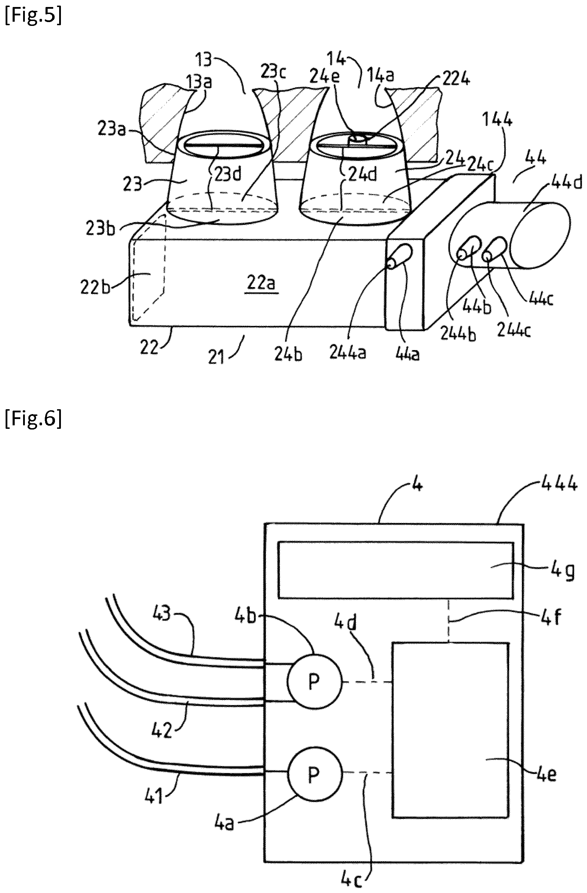

Further, in one of the prongs **23, 24,** a supplementary conduit **24e** is arranged. Said supplementary conduit **24e** comprises an inlet port **224** port located into a second conduit **23c, 24c,** preferably in the vicinity of the free end of the nasal prong **23** in which the supplementary conduit **24e** is arranged, and an outlet port **225** located into the vent conduit **25,** preferably in the vicinity of the venting port **25a.** This supplementary conduit **24e** allows collecting some important physiological parameters, such as the gas pressure in the patient's nostril **14** as explained below in connection with Figure 5.

The nasal sub-system **21** can be made of silicon material so as to be light for the patient **1.** Further, it preferably comprises one or several straps, a headgear or similar a fixing system (not shown) for maintaining the nasal sub-system **21** in position in the nostrils **12, 14** of the patient **1.**

Figure 5 shows the nasal sub-system **21** of Figure 4 to which an additional measurement module **44** has been attached, which can be used for collecting information or data related to the gas flows to and/or from the patient, which can be useful to the medical team.

More precisely, the measurement module **44** comprises a main module body **144** with fixation elements (not shown) for fixing or attaching the measurement module **44** to the nasal sub-system **21.** The main module body **144** comprises a first inner gas conduct fluidly linking or connecting the outlet port **225** to a first port **44a** comprising a first exit orifice **244a** through which the gas can exit the first inner gas conduct and first port **44a** or connection. The first port **44a** is itself fluidly connected to a first flexible tubing **41** for feeding the first flexible tubing **41** with gas.

In the embodiment of Figure 5, the main module body **144** further comprises an expansion portion **44d,** such as a cylindrical part, that is hollow. The lumen or inner volume of the expansion portion **44d** is in fluid communication, on the one end, with the vent conduit **25,** through the outlet port **25a,** and on the other hand, with a second and a third ports **44b, 44c** or connections, that are connected to second and third flexible tubings **42, 43.**

The second and third ports **44b, 44c** comprise second and third exit orifices **244b, 244c,** respectively, for delivering pressurized gas to the second and third flexible tubings **42, 43,** respectively.

The second and third flexible tubings **42, 43** are themselves connected to the processing unit **4,** such as a microcontroller or microprocessor.

The gas travelling into the lumen of the expansion portion **44d** is submitted to a slight pressure drop during its propagation towards the second and third ports **44b, 44c.** This structure constitutes a flow sensor. Gas pressure measurements can be made through the second and third ports **44b, 44c** and then transmitted to the processing unit **4** by the second and third flexible tubings **42, 43** where that can be processed.

Gas pressure measurements made by the module **44** include the instantaneous gas pressure measured in the nostril **14** of the patient **1** as well as the gas pressure before and after any pressure drop into the lumen of the expansion portion **44d.**

Once processed, those data constitute valuable information to the medical team.

Figure 6 represents an embodiment of a processing unit **4** that can be fluidly connected to the nasal sub-system **21** according to the present invention as shown in Figure 5.

The processing unit **4** comprises a casing **444** that embeds two pressure sensors **4a, 4b.**

First sensor **4a** is connected to the first tubing **41** connected to the first port **44a** for thereby monitoring the instantaneous pressure in one of the patient's nostrils **13, 14.** First pressure sensor **4a** is electrically connected with cable **4c** to a controller **4e** which will further transform and process the pressure information. For instance, the average pressure over a given period of time, for example over 10 sec, will help the medical service provider in adjusting the gaseous flow delivered by the High Flow Generator **3** in order to keep the pressure at a safe level in the patient's upper airways.

Controller **4e** can comprise a microprocessor running one or several algorithms.

Useful information may result from the analysis by controller **4e** of the instantaneous pressure provided by the first pressure sensor **4a.** Indeed, upon inhalation, the instantaneous pressure will drop below the average pressure as most of the flow will be inspired by the patient **1.** On the contrary, a slight overpressure will happen upon exhalation of gas by the patient **1** as the total flow will be the sum of the flow coming from the High Flow Generator **3** and the flow exhaled by the patient **1.**

Hence, providing controller **4e** with processing means to process and analyze these pressure swings around the mean pressure value will give access to the patient's respiratory breath, which is also a very valuable indicator for the medical service provider or other members of the medical team.

In the same spirit, the second pressure sensor **4b** is connected to the second and third tubings **42, 43** which are respectively connected to the second and third ports **44b, 44c.** The second pressure sensor **4b** is preferably a differential pressure sensor allowing measuring the slight pressure difference, i.e. pressure drop, that exists between the second and third tubing **42, 43.** The second sensor **4b** is electrically connected with cable **4d** to controller **4e** which will further transform and process the pressure information delivered by the second sensor **4b.**

For instance, based on the geometry of the expansion portion **44d,** controller **4e** is able to determine the absolute value of the flow, relying on a stored lookup table, for translating the differential pressure readings from pressure sensor **4b** to actual gas flow values.

Having a measure of the instantaneous flow, controller **4e** can calculate an average flow over a given period of time, for example over the course of a breath, which will give a good approximation of the continuous flow generated by the High Flow generator **3.**

In order to have all these parameters (such as pressure, respiratory rate, inspiratory volumes and minute volume) accessible to the physician or the medical team, controller **4e** can electronically transfer these information by means of an electric cable **4f** or other data transmission systems (e.g. wireless radio or infrared communication) to a visual interface **4g** or similar data display system.

The processing unit **4** can further embed additional signal systems, such as an acoustic alarm, for alerting the medical team or the user in cases where the processing unit **4** detects that one parameter is out of a given range of threshold values.

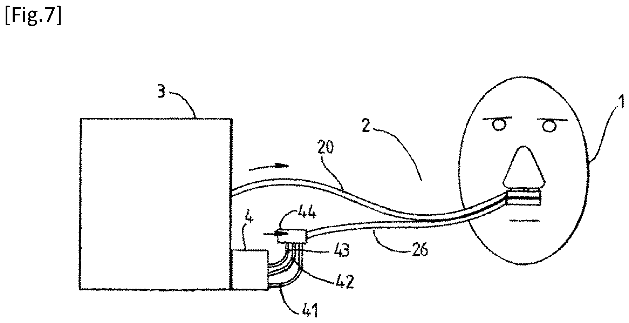

Furthermore, the processing unit **4** can be an independent device (cf. Figure 4) or can be integrated into or attached to the High Flow generator **3** (cf. Figure 7).

Figure 7 shows another embodiment of the nasal sub-system **21** of Figure 4 fluidly connected to an additional measurement module **44** similar to the one of Figure 5 for collecting information or data related to the gas flows to and/or from the patient.

However, in this embodiment, the additional measurement module **44** is not directly attached to the nasal sub-system **21** of Figure 4 but is connected to it by means of one or several conducts **26,** such as a long, flexible hose.

In other words, the additional measurement module **44** is remote from the patient and located close to a processing unit **4,** as the one of Figure 6, to which it is fluidly connected by means of the first, second and third tubings **41, 42, 43** as explained above. Of course, the additional measurement module **44** could be also directly embedded in the processing unit **4** thereby limiting the number of tubings, hoses or similar.

The processing unit **4** can be further attached or plugged to the ventilator **3** as shown in Figure 7 or constitute an independent device electrically linked to the ventilator **3.**

## Claims

1. A gas delivery assembly comprising a nasal sub-system (21) to be connected to a nose of a patient for delivering a respiratory gas to the patient, and a measurement module (44), wherein the nasal sub-system (21), comprises:
- a hollow body (22) with an inner chamber (22a) and an inlet (22b) for receiving a respiratory gas, and
- a pair of nasal prongs (23, 24), in fluid communication with the inner chamber (22a) of the hollow body (22), each nasal prong (23, 24) comprising a pair of inner channels (23b, 23c; 24b, 24c), each pair of inner channels (23b, 23c; 24b, 24c) comprising a first channel (23b; 24b) and a second channel (23c; 24c) arranged in parallel, each first channel (23b; 24b) fluidly connecting in use the internal chamber (22a) of the hollow body (22) with a nostril (13, 14) of the patient (1), and each second channel (23c, 24c) fluidly connecting in use a nostril (13, 14) with a vent conduit (25) arranged in the hollow body (22) and in fluid communication with the atmosphere via at least one venting port (25a),
**characterized in that** a supplementary conduit (24e) is arranged in one of the two prongs (23, 24) of the nasal sub-system (21), said supplementary conduit (24e) comprising an inlet port (224) port located within one of the second channels (23c, 24c) of the nasal prongs (23, 24), and an outlet port (225) located into the vent conduit (25), and
wherein the measurement module (44) comprises a main module body (144) in fluid communication with the nasal sub-system (21), said main module body (144) comprising:
- a first port (44a) comprising a first exit orifice (244a) for a first gas flow from the measurement module (44), and a second and a third port (44b, 44c) comprising a second and a third exit orifice (244b, 244c),
- a first inner gas conduct in fluid communication with the outlet port (225) of the nasal sub-system (21) and further fluidly connected to the first port (44a) of the main module body (144), and
- a hollow expansion portion (44d) comprising an inner volume in fluid communication with the vent conduit (25) of the nasal sub-system (21), and with the second and third ports (44b, 44c) of the main module body (144).

2. The gas delivery assembly according to claim 1, wherein the vent conduit (25) of the hollow body (22) of the nasal sub-system (21) is in fluid communication with the inner chamber (22a) of the hollow body (22) via each pair of inner channels (23b, 23c; 24b, 24c).

3. The gas delivery assembly according to claim 1, wherein an internal separation wall (23d, 24d) arranged in each prong (23, 24) of the nasal sub-system (21) separates the first channel (23b, 24b) from the second channel (23c, 24c) of each prong (23, 24).

4. The gas delivery assembly according to claim 1, wherein the nasal prongs (23, 24) of the nasal sub-system (21) are sized so as to match the inner walls (13a, 14a) of the patient's nostrils (13, 14).

5. The gas delivery assembly according to claim 1, wherein the prongs (23, 24) of the nasal sub-system (21) have a conical or tronconical general shape.

6. The gas delivery assembly according to claim 1, wherein the structure comprising the lumen of the expansion portion (44d) and the second and third ports (44b, 44c) constitutes a flow sensor.

7. The gas delivery assembly according to claim 1, wherein the inlet port (224) of the supplementary conduit (24e) of the nasal sub-system (21) is located in a vicinity of a free end of one of the nasal prongs (23, 24).

8. The gas delivery assembly according to claim 1, wherein the supplementary conduit (24e) of the nasal sub-system (21) comprises an outlet port (225) located in a vicinity of the least one venting port (25a) of the vent conduit (25).

9. The gas delivery assembly according to claim 1, wherein the nasal sub-system (21) further comprises a fixing system for maintaining the nasal prongs (23, 24) in position into the patient's nostrils (13, 14).

10. The gas delivery assembly according to claim 1, wherein the main module body (144) is attached to the nasal sub-system (21).

11. A gas delivery apparatus comprising a gas delivery assembly according to claim 1 and further comprising a high flow generator (3) fluidly connected to the gas delivery assembly for delivering a respiratory gas to a patient.

12. The gas delivery apparatus according to claim 11, further comprising a processing unit (4) fluidly connected to the nasal sub-system (21).

13. The gas delivery apparatus according to claim 11, further comprising a processing unit (4) electrically connected to the high flow generator (3).

14. The gas delivery apparatus according to claim 11, further comprising a processing unit (4) comprising two pressure sensors (4a, 4b) electrically connected to a controller (4e) for providing pressure signals to said controller (4e).

15. The gas delivery apparatus according to claim 14, wherein the processing unit (4) further comprises a visual interface (4g) electrically connected to the controller (4e) for displaying data issued by the controller (4e).

## Patentansprüche

1. Gasabgabeanordnung, umfassend ein Nasen-Untersystem (21), das an eine Nase eines Patienten anzuschließen ist, um ein Atemgas an den Patienten abzugeben, und ein Messmodul (44), wobei das Nasen-Untersystem (21) umfasst:
- einen Hohlkörper (22) mit einer internen Kammer (22a) und einem Einlass (22b) zum Empfangen eines Atemgases, und
- ein Paar von Nasenstutzen (23, 24), in Fluidkommunikation mit der internen Kammer (22a) des Hohlkörpers (22), wobei jeder Nasenstutzen (23, 24) ein Paar von internen Kanälen (23b, 23c; 24b, 24c) umfasst, wobei jedes Paar von internen Kanälen (23b, 23c; 24b, 24c) einen ersten Kanal (23b; 24b) und einen zweiten Kanal (23c; 24c), die parallel angeordnet sind, umfasst, wobei jeder erste Kanal (23b; 24b) im Betrieb die interne Kammer (22a) des Hohlkörpers (22) fluidisch an ein Nasenloch (13, 14) des Patienten (1) anschließt, und jeder zweite Kanal (23c; 24c) im Betrieb ein Nasenloch (13, 14) fluidisch an eine Entlüftungsleitung (25) anschließt, die in dem Hohlkörper (22) angeordnet ist, und über mindestens einen Entlüftungsanschluss (25a) in Fluidkommunikation mit der Atmosphäre ist,
**dadurch gekennzeichnet, dass** eine zusätzliche Leitung (24e) in einem der zwei Stutzen (23, 24) des Nasen-Untersystems (21) angeordnet ist, wobei die zusätzliche Leitung (24e) einen Einlassanschluss (224), wobei sich der Anschluss innerhalb eines der zweiten Kanäle (23c, 24c) der Nasenstutzen (23, 24) befindet, und einen Auslassanschluss (225), der sich in der Entlüftungsleitung (25) befindet, umfasst, und
wobei das Messmodul (44) einen Hauptmodulkörper (144) in Fluidkommunikation mit dem Nasen-Untersystem (21) umfasst, wobei der Hauptmodulkörper (144) umfasst:
- einen ersten Anschluss (44a), umfassend eine erste Ausgangsöffnung (244a) für eine erste Gasströmung von dem Messmodul (44), und einen zweiten und dritten Anschluss (44b, 44c), umfassend eine zweite und eine dritte Ausgangsöffnung (244b, 244c),
- eine erste interne Gasführung in Fluidkommunikation mit dem Auslassanschluss (225) des Nasen-Untersystems (21) und weiter fluidisch an dem ersten Anschluss (44a) des Hauptmodulkörpers (144) angeschlossen, und
- einen hohlen Expansionsabschnitt (44d), umfassend ein internes Volumen in Fluidkommunikation mit der Entlüftungsleitung (25) des Nasen-Untersystems (21) und mit den zweiten und dritten Anschlüssen (44b, 44c) des Hauptmodulkörpers (144).

2. Gasabgabeanordnung nach Anspruch 1, wobei die Entlüftungsleitung (25) des Hohlkörpers (22) des Nasen-Untersystems (21) über jedes Paar von internen Kanälen (23b, 23c; 24b, 24c) in Fluidkommunikation mit der internen Kammer (22a) des Hohlkörpers (22) ist.

3. Gasabgabeanordnung nach Anspruch 1, wobei eine innere Trennwand (23d, 24d), die in jedem Stutzen (23, 24) des Nasen-Untersystems (21) angeordnet ist, den ersten Kanal (23b, 24b) von dem zweiten Kanal (23c, 24c) jedes Stutzens (23, 24) trennt.

4. Gasabgabeanordnung nach Anspruch 1, wobei die Nasenstutzen (23, 24) des Nasen-Untersystems (21) bemessen sind, um in die internen Wände (13a, 14a) der Nasenlöcher des Patienten (13, 14) zu passen.

5. Gasabgabeanordnung nach Anspruch 1, wobei die Stutzen (23, 24) des Nasen-Untersystems (21) eine konische oder tronkonische allgemeine Form aufweisen.

6. Gasabgabeanordnung nach Anspruch 1, wobei die Struktur, die das Lumen des Expansionsabschnitts (44d) und die zweiten und dritten Anschlüsse (44b, 44c) umfasst, einen Durchsatzsensor bildet.

7. Gasabgabeanordnung nach Anspruch 1, wobei sich der Einlassanschluss (224) der zusätzlichen Leitung (24e) des Nasen-Untersystems (21) in einer Nähe eines freien Endes von einer der Nasenstutzen (23, 24) befindet.

8. Gasabgabeanordnung nach Anspruch 1, wobei die zusätzliche Leitung (24e) des Nasen-Untersystems (21) einen Auslassanschluss (225) umfasst, der sich in einer Nähe des mindestens einen Entlüftungsanschlusses (25a) der Entlüftungsleitung (25) befindet.

9. Gasabgabeanordnung nach Anspruch 1, wobei das Nasen-Untersystem (21) weiter ein Befestigungssystem zum Halten der Nasenstutzen (23, 24) in Position in den Nasenlöchern des Patienten (13, 14) umfasst.

10. Gasabgabeanordnung nach Anspruch 1, wobei der Hauptmodulkörper (144) an dem Nasen-Untersystem (21) angebracht ist.

11. Gasabgabeeinrichtung, umfassend eine Gasabgabeanordnung nach Anspruch 1, und weiter einen High-Flow-Generator (3) umfassend, der zur Abgabe eines Atemgases an einen Patienten fluidisch an die Gasabgabeanordnung angeschlossen ist.

12. Gasabgabeeinrichtung nach Anspruch 11, weiter eine Verarbeitungseinheit (4) umfassend, die fluidisch an das Nasen-Untersystem (21) angeschlossen ist.

13. Gasabgabeeinrichtung nach Anspruch 11, weiter eine Verarbeitungseinheit (4) umfassend, die elektrisch an den High-Flow-Generator (3) angeschlossen ist.

14. Gasabgabeeinrichtung nach Anspruch 11, weiter eine Verarbeitungseinheit (4) umfassend, die zwei Drucksensoren (4a, 4b) umfasst, die elektrisch an eine Steuerung (4e) angeschlossen sind, um der Steuerung (4e) Drucksignale bereitzustellen.

15. Gasabgabeeinrichtung nach Anspruch 14, wobei die Verarbeitungseinheit (4) weiter eine visuelle Schnittstelle (4g) umfasst, die elektrisch an die Steuerung (4e) angeschlossen ist, um durch die Steuerung (4e) ausgegebene Daten anzuzeigen.

## Revendications

1. Ensemble de délivrance de gaz comprenant un sous-système nasal (21) à relier au nez d'un patient pour délivrer un gaz respiratoire au patient, et un module de mesure (44), dans lequel le sous-système nasal (21) comprend :
- un corps creux (22) avec une chambre intérieure (22a) et une entrée (22b) pour recevoir un gaz respiratoire, et
- un couple de canules nasales (23, 24), en communication fluidique avec la chambre intérieure (22a) du corps creux (22), chaque canulenasale (23, 24) comprenant un couple de canaux intérieurs (23b, 23c ; 24b, 24c), chaque couple de canaux intérieurs (23b, 23c ; 24b, 24c) comprenant un premier canal (23b ; 24b) et un second canal (23c ; 24c) agencés en parallèle, chaque premier canal (23b ; 24b) reliant fluidiquement , lors de l'utilisation, la chambre interne (22a) du corps creux (22) à une narine (13, 14) du patient (1), et chaque second canal (23c, 24c) reliant fluidiquement, lors de l'utilisation, une narine (13, 14) à un conduit de ventilation (25) agencé dans le corps creux (22) et en communication fluidique avec l'atmosphère via au moins un port de ventilation (25a),
**caractérisé en ce qu'**un conduit supplémentaire (24e) est agencé dans une des deux canules (23, 24) du sous-système nasal (21), ledit conduit supplémentaire (24e) comprenant un port d'entrée (224), port situé à l'intérieur de l'un des seconds canaux (23c, 24c) des canules nasales (23, 24), et un port de sortie (225) situé dans le conduit de ventilation (25), et
dans lequel le module de mesure (44) comprend un corps de module principal (144) en communication fluidique avec le sous-système nasal (21), ledit corps de module principal (144) comprenant :
- un premier port (44a) comprenant un premier port de sortie (244a) pour un premier flux de gaz en provenance du module de mesure (44) et un deuxième et un troisième port (44b, 44c) comprenant un deuxième et un troisième port de sortie (244b, 244c),
- un premier conduit de gaz intérieur en communication fluidique avec le port de sortie (225) du sous-système nasal (21) et relié en outre fluidiquement au premier port (44a) du corps de module principal (144), et
- une portion d'expansion creuse (44d) comprenant un volume intérieur en communication fluidique avec le conduit de ventilation (25) du sous-système nasal (21) et avec les deuxième et troisième ports (44b, 44c) du corps de module principal (144).

2. Ensemble de délivrance de gaz selon la revendication 1, dans lequel le conduit de ventilation (25) du corps creux (22) du sous-système nasal (21) est en communication fluidique avec la chambre intérieure (22a) du corps creux (22) via chaque couple de canaux intérieurs (23b, 23c ; 24b, 24c).

3. Ensemble de délivrance de gaz selon la revendication 1, dans lequel une paroi de séparation interne (23d, 24d) agencée dans chaque canule (23, 24) du sous-système nasal (21) sépare le premier canal (23b, 24b) du second canal (23c, 24c) de chaque canule (23, 24).

4. Ensemble de délivrance de gaz selon la revendication 1, dans lequel les canules nasales (23, 24) du sous-système nasal (21) sont dimensionnées afin de correspondre aux parois intérieures (13a, 14a) des narines du patient (13, 14).

5. Ensemble de délivrance de gaz selon la revendication 1, dans lequel les canules (23, 24) du sous-système nasal (21) ont une forme générale conique ou tronconique.

6. Ensemble de délivrance de gaz selon la revendication 1, dans lequel la structure comprenant la lumière de la portion d'expansion (44d) et les deuxième et troisième ports (44b, 44c) constitue un capteur de flux.

7. Ensemble de délivrance de gaz selon la revendication 1, dans lequel le port d'entrée (224) du conduit supplémentaire (24e) du sous-système nasal (21) est situé au voisinage d'une extrémité libre d'une des canules nasales (23, 24).

8. Ensemble de délivrance de gaz selon la revendication 1, dans lequel le conduit supplémentaire (24e) du sous-système nasal (21) comprend un port de sortie (225) situé au voisinage de l'au moins un port de ventilation (25a) du conduit de ventilation (25).

9. Ensemble de délivrance de gaz selon la revendication 1, dans lequel le sous-système nasal (21) comprend en outre un système de fixation pour maintenir les canules nasales (23, 24) en position dans les narines du patient (13, 14).

10. Ensemble de délivrance de gaz selon la revendication 1, dans lequel le corps de module principal (144) est attaché au sous-système nasal (21).

11. Appareil de délivrance de gaz comprenant un ensemble de délivrance de gaz selon la revendication 1 et comprenant en outre un générateur de flux élevé (3) relié fluidiquement à l'ensemble de délivrance de gaz pour délivrer un gaz respiratoire à un patient.

12. Appareil de délivrance de gaz selon la revendication 11, comprenant en outre une unité de traitement (4) reliée fluidiquement au sous-système nasal (21).

13. Appareil de délivrance de gaz selon la revendication 11, comprenant en outre une unité de traitement (4) électriquement reliée au générateur de flux intense (3).

14. Appareil de délivrance de gaz selon la revendication 11, comprenant en outre une unité de traitement (4) comprenant deux capteurs de pression (4a, 4b) électriquement reliés à une unité de commande (4e) pour fournir des signaux de pression à ladite unité de commande (4e).

15. Appareil de délivrance de gaz selon la revendication 14, dans lequel l'unité de traitement (4) comprend en outre une interface visuelle (4g) électriquement reliée à l'unité de commande (4e) pour afficher des données délivrées par l'unité de commande (4e).
